# EUROPEAN PATENT APPLICATION

(11) **EP 1 964 848 A1**
(43) Date of publication of application: **03.09.2008**
(21) Application number: 07090035.2
(22) Date of filing: 01.03.2007
(51) Int. Cl.: C07K 5/023, C07K 1/13, C07D 249/18

(54) **Radiofluorination methods**

(71) Applicant: Bayer Schering Pharma Aktiengesellschaft, 13353 Berlin (DE)
(72) Inventor: Srinivasan, Anath, 10629 Berlin (DE); Stellfeld, Timo, 14197 Berlin (DE)

(57) **Abstract**

The present invention relates to radiolabelled substitute benzene compounds for diagnostic imaging. The present invention provides methods for preparation of such compounds, in particular, preparation of novel compounds which serve as precursors for ¹⁸F-labeling, and the use of thus ¹⁸F-labeled compounds for diagnostic imaging.

## Description

### Field of invention:

This invention relates to novel substitute benzene compounds, which provide access to ^{1B}F-labelled compounds, methods for the preparation of such compounds and their use for diagnostic imaging.

### Background

The nucleophilic aromatic ¹⁸F-fluorination reaction is of great importance for ¹⁸F-labelled radiopharmaceuticals which are used as *in vivo* imaging agents targeting and visualizing diseases, e.g. solid tumours.

The conversions of *mono-* (mainly para-) substituted phenyl-trimethylammonium derivatives to substituted [¹⁸F]-fluorobenzene derivatives which serve as radiopharmaceutical itself or as prosthetic group for the ¹⁸F-labeling of small and large molecules have been reported in the literature (Irie et al. 1982, Fluorine Chem., 27, (1985), 117-191; Haka et al. 1989) (see scheme 1).

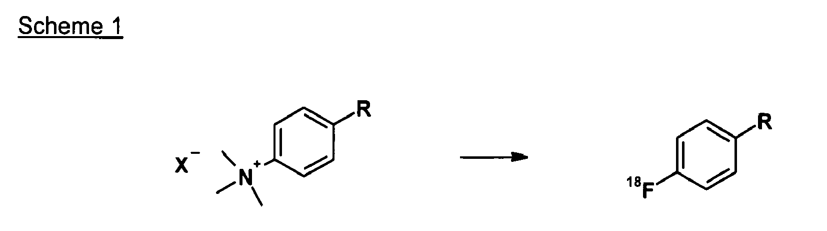

There are only a few publications about nucleophilic aromatic ¹⁸F-fluorination reactions of trimethylammonium-substituted aromatic derivatives which contain two or more substituents beside the trimethylammonium moiety:
Oya et al. treated [2-Chloro-5-(2-dimethylcarbamoyl-phenylsulfanyl)-4-nitro-phenyl]-trimethylammonium triflate with [18F] potassium fluoride and obtained the desired 18F-labelled compound (Journal of Medicinal Chemistry (2002), 45(21), 4716-4723).
Li et al. reported on the 18F-fluorination reaction of 4-(N,N,N-trimethylammonium)-3-cyano-3'-iodobenzophenone triflate (Bioconjugate Chemistry (2003), 14(2), 287-294).
Enas et al. converted (2,2-Dimethyl-1,3-dioxo-indan-5-yl)-trimethylammonium triflate into the desired 18F-labelled compound (Journal of Fluorine Chemistry (1993), 63(3), 233-41).
Seimbille et al. and other groups labelled (2-Chloro-4-nitro-phenyl)-trimethylammonium triflate successfully with 18F (J. Labelled Compd. Radiopharm., (2005), 48, 11, 829-843).
(2-Benzyloxy-4-formyl-phenyl)-trimethylammonium triflate was successfully labelled with ¹⁸F at high temperature (130° C) by Langer et. al. (Bioorg. Med. Chem.; EN; 9; 3; 2001; 677 - 694).
Lang et al. radiolabelled trimethyl-(2-methyl-4-pentamethylphenyl methoxycarbonyl-phenyl)-ammonium triflate by use of [18F] potassium fluoride (J. Med. Chem., 42; 9; 1999; 1576 - 1586).
Trimethyl-(4-nitro-naphthalen-1-yl)-ammonium triflate was labelled with ¹⁸F by Amokhtari et al. (J. Labelled Compd. Radiopharm.; S42; 1; (1999); S622 - S623).
Lemaire et al. converted (2-formyl-5-methoxy-phenyl)-trimethylammonium triflate into the desired 18F-labelled product (J. Labelled Compd. Radiopharm.; 44; 2001; S857 - S859).
VanBrocklin et al. described the 18F labeling of (2-bromo-4-nitro-phenyl)-trimethylammonium triflate (J. Labelled Compd. Radiopharm., 44; 2001; S880 - S882).

Cetir Centre Medic reported on the successful ¹⁸F-labeling of (5-Chloro-8-hydroxy-quinolin-7-yl)-trimethylammonium triflate (EP1563852 A1).
Most of these mentioned ¹⁸F-labelled aromatic derivatives which contain two or more additional substituents can not be coupled to chemical functionalities like amines, thiols, carboxylic acids, phenols or other chemicals groups of complex molecules like peptides without further transformations.

¹⁸F-labelings of more complex radiopharmaceuticals like peptides take place in all known publications in a two- or multi-step strategy (see scheme 2, overview: Eur. J. Nucl. Med. (2001), 28, 929-938).

For these kinds of ¹⁸F-labeling also mono-substituted phenyl-trimethylammonium derivatives are used and react in a first step with [¹⁸F] potassium fluoride to obtain substituted [¹⁸F]-fluorobenzene derivatives. These compounds are then coupled in a second step to larger and more complex molecules like peptides or nucleotides (see scheme 2).

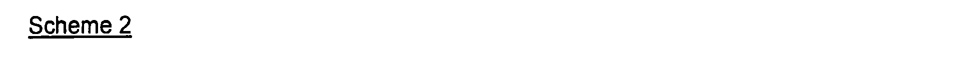

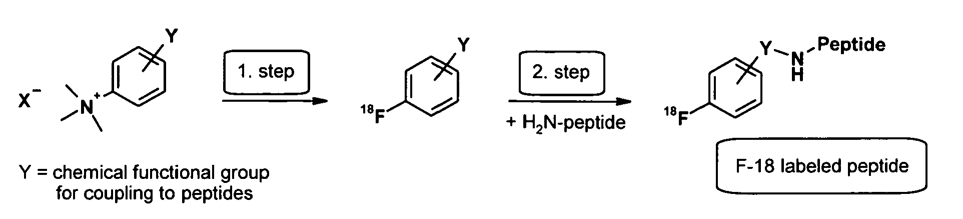

Especially 4-[¹⁸F]fluorobenzaldehyde has been used in many examples for F-18 labeling of complex molecules (e.g. Journal of Nuclear Medicine (2004), 45(5), 892-902). But also N-succinimidyl-8-[4'-[¹⁸F]fluorobenzylamino]suberate (Bioconjugate Chem., (1991), 2, 44-49), 4-[¹⁸F]fluorophenacyl bromide and 3-[¹⁸F]fluoro-5-nitrobenzimidate (J. Nucl. Med., (1987), 28, 462-470), m-maleimido-N-(p-[¹⁸F]fluorobenzyl)-benzamide (J. Labelled Compd. Radiopharm (1989), 26, 287-289,), *N*-{4-[4-[¹⁸F]fluorobenzylidene(aminooxy)-butyl}-maleimide (Bioconjugate Chem., (2003), 14, 1253-1259), [¹⁸F]*N*-(4-fluorobenzyl)-2-bromoacetamide (Bioconjugate Chem., (2000), 11, 627-636) and [¹⁸F]-3,5-difluorophenyl azide (and 5 derivatives) (J. Org. Chem., (1995), 60, 6680-6681) are known examples. F-18 labeling of peptides via para-[¹⁸F]-fluorobenzoates is also a very common method either by coupling of the corresponding acid with additional activating agents (such as 1,3-dicyclohexylcarbodiimide/1-hydroxy-7-azabenzotriazole (DCC/HOAt) or N-[(dimethylamino)-1H-1,2,3-triazolyl[4,5]pyridine-1-yl-methylene]-N-methyl-methan-aminium hexafluorophosphate N-oxide (HATU/DIPEA, Eur. J. Nucl. Med. Mol. Imaging., (2002), 29, 754-759) or by isolated N-succinimidyl 4-[¹⁸F]fluorobenzoate (Nucl. Med. Biol. (1996), 23, 365).

As outlined above, the current state of art provides the trimethylammonium group and the nitro group as the sole leaving groups to afford ¹⁸F-labelled compounds for both indirect labeling of peptides via prosthetic groups (references above), direct labeling of peptides as well as for small molecules (see EP06090166) not published at the date of filing.

### Further references:

WO2004/080492 A1, "Methods of radiofluorination of biologically active vectors" Published 23 September 2004.

Bruus-Jensen K, Poethko T, Schottelius M, Hauser A, Schwaiger M, Wester H. J." Chemoselective hydrazones formation between HYNIC-functionalized peptides and (18)F-fluorinated aldehydes." Nucl Med Biol.(2006) 33(2):173-83

Poethko T, Schottelius M, Thumshirn G, Hersel U, Herz M, Henriksen G, Kessler H, Schwaiger M, Wester H. J."Two-step methodology for high-yield routine radiohalogenation of peptides: (18)F-labelled RGD and octreotide analogs." J Nucl Med. 2004 May;45(5):892-902 and references therein.

Zhang X, Cai W, Cao F, Schreibmann E, Wu Y, Wu J.C, Xing L, Chen X. "18F-labelled bombesin analogs for targeting GRP receptor-expressing prostate cancer." J Nucl. Med. (2006), 47(3):492-501.

Li Z, Ding Y. S, Gifford A, Fowler J. S, Gatley J. S."Synthesis of structurally identical fluorine-18 and iodine isotope labeling compounds for comparative imaging" Bioconjug Chem. (2003),14(2):287-94.

For a number of these diagnostic imaging compounds it would be detrimental for their targeting activity to be subject to harsh reaction conditions during radiolabeling like e.g. high temperatures which are usually used during nucleophilic aromatic ¹⁸F-fluorination reaction. That is why in the prior art e.g. peptides are labelled via a two step approach as outlined above. This two step approach is time consuming and requires multiple purification steps. Displacement of the trimethylammonium and/or nitro leaving groups is accomplished at elevated temperatures and hence it is desirable to provide alternative leaving groups to accomplish the ¹⁸F incorporation under milder conditions compatible with chemical and biological stability of the targeting agent. Due to the limited half life of the ¹⁸F isotope of about only 111 minutes, there is a high need for compounds and methods that allow provision of the ¹⁸F-radiolabelled compound with less steps needed.

The problem to be solved by the present invention is the provision of compounds and methods that allow for radiolabeling compounds with ¹⁸F in a one-step approach.

### Summary of the Invention

The present invention provides novel substitute benzene compounds of Formulae I and II. Compounds of Formula I can be converted into compounds of Formula II by means of a one-step radiolabeling reaction with ¹⁸F.

Further, the invention provides a one-step method of radiofluorinating compounds according to Formula I in order to arrive at compounds according to Formula II.

The present invention also provides diagnostic compositions comprising a compound of Formula I or a radiolabelled compound of formula II and a pharmaceutically acceptable carrier or diluent.

The invention further provides a method of imaging diseases, the method comprising introducing into a patient a detectable quantity of a labelled compound of Formula II or pharmaceutically acceptable salt, ester, amide or prodrug thereof.

The invention provides the compounds of Formula II for use as medicament.

Another aspect of the invention is directed to the use of compounds of Formula I or II for the manufacture of a medicament.

The present invention also provides a kit for preparing a radiopharmaceutical preparation, said kit comprising a sealed vial containing a predetermined quantity of the compound of Formula I.

A further aspect of this invention is directed to methods and intermediates useful for synthesizing the tumor imaging compounds of Formula I and II described herein.

More specifically the compounds of this invention are useful for the imaging of a variety of cancers including but not limited to: carcinoma such as bladder, breast, colon, kidney, liver, lung, including small cell lung cancer, esophagus, gall-bladder, ovary, pancreas, stomach, cervix, thyroid, prostate, and skin, hematopoetic tumors of lymphoid and myeloid lineage, tumors of mesenchymal origin, tumors of central peripheral nervous systems, other tumors, including melanoma, seminoma, teratocarcinoma, osteosarcoma, xeroderma pigmentosum, keratoxanthoma, thyroid follicular cancer, and Karposi's sarcoma.

### Details of the Invention

In a first aspect the present invention is directed to compounds of Formula I

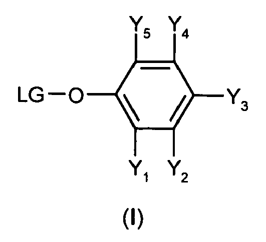

Y₁, Y₂, Y₃, Y₄, and Y₅ are independently from each other H, CN, Cl, F, CF₃, NO₂, COCH₃, or SO₂CH₃, with the proviso that exactly one residue of Y₁, Y₂, Y₃, Y₄, and Y₅ is A-B-D-P, wherein
- A: is CO or -SO₂,
- B: is NH or NR, wherein R is a branched, cyclic or linear C₁ to C₆ alkyl group, preferably CH₃ or C₂H₅,
- D: is (CH₂)ₚ-CO with p being an integer between 1 and 10, or (CH₂-CH₂-O)_{q}-CH₂-CH₂-CO with q being an integer between 1 and 5,
- B-D: together is a bond or one amino acid residue or an amino acid sequence with two (2) to twenty (20) amino acid residues,
- P: is a targeting agent,
- LG: is a leaving group, suitable for displacement by means of a nucleophilic aromatic substitution reaction
and pharmaceutically acceptable salt, hydrate or solvate thereof.

In preferred embodiments Y₁, Y₂, Y₃, Y₄, and Y₅ are independently from each other selected from H, CN and Cl. In a more preferred embodiment Y₁ or Y₅ are independently from each other CN or Cl

In a more preferred embodiment one of Y₁ or Y₅ is CN or Cl.

In preferred embodiments A is CO.

In preferred embodiments B is selected from NH and NCH₃.

In preferred embodiments D is selected from (CH₂)₄-CO.

For the purpose of the present invention the term "amino acid sequence" is defined herein as a polyamide obtainable by (poly)condensation of at least two amino acids. For the purpose of the present invention the term "amino acid" means any molecule comprising at least one amino group and at least one carboxyl group. In other words, an amino acid is a molecule that has a carboxylic acid functionality and an amine nitrogen having at least one free hydrogen, preferably in alpha position thereto. Thus, a dipeptide having a free amino group at the N-terminus and a free carboxyl group at the C-terminus is not to be considered as a single "amino acid" in the above definition. The amide bond between two adjacent amino acid residues which is obtained from such a condensation is defined as "peptide bond". Optionally, the nitrogen atoms of the polyamide backbone (indicated as NH above) may be independently alkylated, e.g. with C₁-C₆-alkyl, preferably -CH₃.

For the purpose of the specification an amino acid residue is derived from the corresponding amino acid by forming a peptide bond with another amino acid.

For the purpose of the specification an amino acid sequence may comprise naturally occurring and/or artificial amino acid residues, proteinogenic and/or non-proteinogenic amino acid residues. The non-proteinogenic amino acid residues may be further classified as (a) homo analogues of proteinogenic amino acids, (b) β-homo analogues of proteinogenic amino acid residues and (c) further non-proteinogenic amino acid residues.

Accordingly, the amino acid residues are derived from the corresponding amino acids, e.g. from
- proteinogenic amino acids, namely Ala, Arg, Asn, Asp, Cys, Gln, Glu, Gly, His, Ile, Leu, Lys, Met, Phe, Pro, Ser, Thr, Trp, Tyr and Val; or
- non-proteinogenic amino acids, such as
   o homo analogues of proteinogenic amino acids wherein the sidechain has been extended by a methylene group, e.g. Homoalanine (Hal), Homoarginine (Har), Homocysteine (Hcy), Homoglutamine (Hgl), Homohistidine (Hhi), Homoisoleucine (Hil), Homoleucine (Hle), Homolysine (Hly), Homomethionine (Hme), Homophenylalanine (Hph), Homoproline (Hpr), Homoserine (Hse), Homothreonine (Hth), Homotryptophane (Htr), Homotyrosine (Hty) and Homovaline (Hva);
   o β-homo analogues of proteinogenic amino acids wherein a methylene group has been inserted between the β-carbon and the carboxyl group yielding β-amino acids, e.g. β-Homoalanine (βHal), β-Homoarginine (βHar), β-Homoasparagine (βHas), β-Homocysteine (βHcy), β-Homoglutamine (βHgl), β-Homohistidine (βHhi), β-Homoisoleucine (βHil), β-Homoleucine (βHle), β-Homolysine (βHly), β -Homomethionine (βHme), β-Homophenylalanine (βHph), β-Homoproline (βHpr), β-Homoserine (βHse), β-Homothreonine (βHth), β-Homotryptophane (βHtr), β -Homotyrosine (βHty) and β-Homovaline (βHva);
   ο further non-proteinogenic amino acids, e.g. α-Aminoadipic acid (Aad), β -Aminoadipic acid (βAad), α-Aminobutyric acid (Abu), α-Aminoisobutyric acid (Aib), β-Alanine (βAla), 4-Aminobutyric acid (4-Abu), 5-Aminovaleric acid (5-Ava), 6-Aminohexanoic acid (6-Ahx), 8-Aminooctanoic acid (8-Aoc), 9-Aminononanoic acid (9-Anc), 10-Aminodecanoic acid (10-Adc), 12-Aminododecanoic acid (12-Ado), α-Aminosuberic acid (Asu), Azetidine-2-carboxylic acid (Aze), β-Cyclohexylalanine (Cha), Citrulline (Cit), Dehydroalanine (Dha), χ-Carboxyglutamic acid (Gla), α-Cyclohexylglycine (Chg), Propargylglycine (Pra), Pyroglutamic acid (Glp), α-tert-Butylglycine (Tle), 4-Benzoylphenylalanine (Bpa), δ-Hydroxylysine (Hyl), 4-Hydroxyproline (Hyp), allo-Isoleucine (alle), Lanthionine (Lan), (1-naphthyl)alanine (1-Nal), (2-naphthyl)alanine (2-Nal), Norleucine (Nle), Norvaline (Nva), Ornithine (Orn), Phenylglycin (Phg), Pipecolic acid (Pip), Sarcosine (Sar), Selenocysteine (Sec), Statine (Sta), β-Thienylalanine (Thi), 1,2,3,4-Tetrahydroisochinoline-3-carboxylic acid (Tic), allo-Threonine (aThr), Thiazolidine-4-carboxylic acid (Thz), γ-Aminobutyric acid (GABA), iso-Cysteine (iso-Cys), Diaminopropionic acid (Dpr), 2,4-Diaminobutyric acid (Dab), 3,4-Diaminobutyric acid (γβDab), Biphenylalanine (Bip), Phenylalanine substituted in para-position with -C₁-C₆-alkyl, -halide, -NH₂, -CO₂H or Phe(4-R) (wherein R = -C₁-C₆-alkyl, -halide, -NH₂, or -CO₂H); peptide nucleic acids (PNA, cf. P.E. Nielsen, Acc.Chem.Res. 32, 624-30)
- or their N-alkylated analogues, such as their N-methylated analogues.

Cyclic amino acids may be proteinogenic or non-proteinogenic, such as Pro, Aze, Glp, Hyp, Pip, Tic and Thz.

For further examples and details reference can be made to e.g. J.H. Jones, J. Peptide Sci. 2003, 9, 1-8 which is incorporated herein by reference.

The terms "non-proteinogenic amino acid" and "non-proteinogenic amino acid residue" also encompasses derivatives of proteinogenic amino acids. For example, the side chain of a proteinogenic amino acid residue may be derivatized thereby rendering the proteinogenic amino acid residue "non-proteinogenic". The same applies to derivatives of the C-terminus and/or the N-terminus of a proteinogenic amino acid residue terminating the amino acid sequence.

For the purpose of the specification a proteinogenic amino acid residue is derived from a proteinogenic amino acid selected from the group consisting of Ala, Arg, Asn, Asp, Cys, Gln, Glu, Gly, His, Ile, Leu, Lys, Met, Phe, Pro, Ser, Thr, Trp, Tyr and Val either in L- or D-configuration; the second chiral center in Thr and Ile may have either R- or S-configuration. Therefore, for example, any posttranslational modification of an amino acid sequence, such as N-alkylation, which might naturally occur renders the corresponding modified amino acid residue "non-proteinogenic", although in nature said amino acid residue is incorporated in a protein. Preferably modified amino acids are selected from N-alkylated amino acids, β-amino acids, γ-amino acids, lanthionines, dehydro amino acids, and amino acids with alkylated guanidine moieties.

P is a targeting agent.

For the purposes of this invention, the term "targeting agent" shall have the following meaning: The targeting agent is a compound or moiety that targets or directs the radionuclide attached to it to a specific site in a biological system. A targeting agent can be any compound or chemical entity that binds to or accumulates at a target site in a mammalian body, i.e., the compound localizes to a greater extent at the target site than to surrounding tissue. Preferably the targeting agent is a peptide, or oligonucleotide, particularly one which has specificity to target the complex to a specific site in a biological system. Smaller organic molecules effective for targeting certain sites in a biological system can also be used as the targeting agent. Smaller organic molecules are preferably selected from those described in the following references: Jager P. L, Korte M. A, Lub-de Hooge M. N, van Waarde A, Koopmans K. P, Perik P. J, and de Vries E. G. E, Cancer Imaging (2005) 5, 27-32; Heiss W.D, and Herholz K, J. Nucl. Med. (2006) 47(2), 302-312; and Higuchi T, and Schwaiger M, Curr. Cardiol. Rep. (2006) 8(2), 131-138; Examples for peptides as targeting agent are, but are not limited to, somatostatin and derivatives thereof and related peptides, neuropeptide Y and derivatives thereof and related peptides, gastrin, gastrin releasing peptide, derivatives thereof and related peptides, epidermal growth factor (EGF of various origin), insulin growth factor (IGF) and IGF-1, LHRH agonists and antagonists, transforming growth factors, particularly TGF-α, angiotensin, cholecystokinin (CCK) and analogs; neurotensin and analogs, thyrotropin releasing hormone, prolactin, tumor necrosis factor, IL-1, IL-2, IL-4 or IL-6, interferons, VIP and related peptides, PACAP and related peptides. Such peptides comprise an amino acid sequence from 4 to 100 amino acids.

In preferred embodiments P is selected from peptides comprising an amino acid sequence from 4 to 100 amino acids or oligonucleotides comprising from 4 to 100 nucleotides or peptidomimetics.

In further preferred embodiments P stands for a peptide comprising an amino acid sequence from 4 to 100 amino acids.

In preferred embodiments of compounds of Formula I, P is -NR⁴-peptide, -(CH₂)ₙ-peptide, -NR⁴-small-molecule or -(CH₂)ₙ-small molecule, -NR⁴-oligonucleotide or - (CH₂)ₙ-oligonucleotide and n is an integer between 1 and 6.

In further preferred embodiments of compounds of Formula I, P is -NR⁴-peptide, - (CH₂)ₙ-peptide, and n is an integer between 1 and 6.

In other preferred embodiments of compounds of Formula I, P is , -NR⁴-oligonucleotide or -(CH₂)ₙ-oligonucleoptide and n is an integer between 1 and 6.

In preferred embodiments of compounds of Formula I, P is -NR⁴-small-molecule or-(CH₂)n-small molecule, and n is an integer between 1 and 6.

In preferred embodiments of compounds of Formula I, the leaving group (LG) is selected from the group of 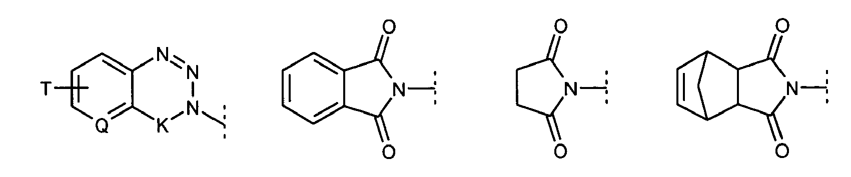 wherein,
- T: is H, or Cl,
- Q: is CH, or N,
- K: is absent, or C=O

In a more preferred embodiments LG is selected from

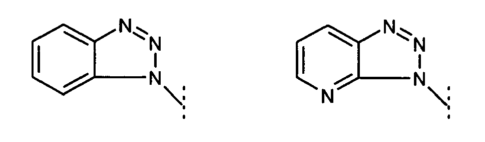

The compound according to Formula I serves as precursor of the compound according to Formula II, wherein the leaving group LG is replaced in a labeling reaction with a ¹⁸F or ¹⁹F atom.

In a second aspect the invention is directed to compounds according to Formula II, 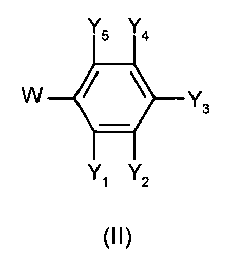 wherein the residues and substituents Y₁, Y₂, Y₃, Y₄, and Y₅ have the same meaning as depicted above for Formula I. This includes in particular all preferred embodiments mentioned above with regard to the residues and substituents Y₁, Y₂, Y₃, Y₄, and Y₅, A, B, D, and P,
W is ¹⁸F or ¹⁹F,
and pharmaceutically acceptable salt, hydrate or solvate thereof.

In preferred embodiments W is ¹⁸F.

In preferred embodiment of compounds of Formula II, Y₁, Y₂, Y₃, Y₄, and Y₅ are independently from each other selected from H, CN and Cl.

In a more preferred embodiment Y₁, Y₂, Y₃, Y₄, and Y₅ are independently from each other CN or.

In a third aspect, the invention is directed to methods for obtaining compound of formula (II) comprising the step of coupling a compound of formula I with ¹⁸F or ¹⁹F and thereafter converting the compound of formula II into a pharmaceutically acceptable salt, hydrate or solvate thereof if desired.

In a preferred embodiment of the method, wherein the compound of formula I is any preferred compound described above for obtaining any preferred compound of formula II described above.

In a fourth aspect, the invention is directed to a composition comprising a compound according to Formula I or II and a pharmaceutically acceptable carrier or diluent.

In a fifth aspect, the invention is directed to a kit comprising a sealed vial containing a predetermined quantity of a compound according to Formula I.

In a sixth aspect, the invention is directed to a compound according to Formula I or II for use as medicament or as diagnostic imaging agent. More preferably the use as imaging agent is for positron emission tomography (PET).

In a seventh aspect, the invention is directed to the use of a compound according to Formula I or II for the manufacturing of a medicament or a diagnostic imaging agent. In a preferred embodiment the use concerns a medicament or a diagnostic imaging agent for treatment or positron emission tomography (PET) imaging respectively. In a more preferred embodiment, the use for imaging tissue at target site by the targeting agent.

The compounds according to Formula I and II of the invention can be chemically synthesized in vitro. In case P is selected to be a peptide, such peptides can generally advantageously be prepared on an amino acid synthesizer. Preferably, particularly when B-D is a sequence of amino acids and P is a peptide and both together are forming a fusion peptide, said fusion peptide may be synthesized sequentially, i.e. the part comprising the amino acid sequence B-D and the targeting agent P may be obtained by subsequently adding suitable activated and protected amino acid derivatives or preformulated amino acid sequences to the growing amino acid chain. For details regarding peptide synthesis it can be referred to e.g. B. Gutte "Peptides: Synthesis, Structures, and Applications", Academic Press, 1995; X.C. Chan et al. "Fmoc Solid Phase Peptide Synthesis: A Practical Approach", Oxford University Press, 2000; J. Jones "Amino Acid and Peptide Synthesis", 2nd ed., Oxford University Press, 2002; M. Bodanszky et al., "Principles of Peptide Synthsis", 2nd ed., Springer, 1993.

The invention also relates to a composition comprising the compound according to Formula I or II of the invention as defined above and a pharmaceutically acceptable carrier.

The radioactively labelled compounds according to Formula II provided by the invention may be administered intravenously with any pharmaceutically acceptable carrier, e.g. conventional medium such as an aqueous saline medium, or in blood plasma medium, as a pharmaceutical composition for intravenous injection. Such medium may also contain conventional pharmaceutical materials such as, for example, pharmaceutically acceptable salts to adjust the osmotic pressure, buffers, preservatives and the like. Among the preferred media are normal saline and plasma. Suitable pharmaceutical acceptable carriers are known to the person skilled in the art. In this regard reference can be made to e.g. Remington's Practice of Pharmacy, 11^{th} ed.

The concentration of the compound according to Formula II and the pharmaceutically acceptable carrier, for example, in an aqueous medium, varies with the particular field of use. A sufficient amount is present in the pharmaceutically acceptable carrier when satisfactory visualization of the imaging target (e.g. a tumor) is achievable.
In accordance with the invention, the radiolabelled compounds according to Formula II either as a neutral complex or as a salt with a pharmaceutically acceptable counterion are administered in a single unit injectable dose. Any of the common carriers known to those with skill in the art, such as sterile saline solution or plasma, can be utilized after radiolabeling for preparing the injectable solution to diagnostically image various organs, tumors and the like in accordance with the invention. Generally, the unit dose to be administered for a diagnostic agent has a radioactivity of about 0.1 mCi to about 100 mCi, preferably 1 mCi to 20 mCi. For a radiotherapeutic agent, the radioactivity of the therapeutic unit dose is about 10 mCi to 700 mCi, preferably 50 mCi to 400 mCi. The solution to be injected at unit dosage is from about 0.01 ml to about 30 ml. For diagnostic purposes after intravenous administration, imaging of the organ or tumor in vivo can take place in a matter of a few minutes. Preferably, imaging takes place between two minutes and two hours, after injecting into patients. In most instances, a sufficient amount of the administered dose will accumulate in the area to be imaged within about 0.1 of an hour to permit the taking of scintigraphic images. Any conventional method of scintigraphic imaging for diagnostic purposes can be utilized in accordance with this invention.

In general, compounds according to Formula II can be generated from compounds according to Formula I by labeling compounds according to Formula I with ¹⁸F or ¹⁹F. Methods and conditions for such labeling reactions are well known to the skilled person (Wüst, F, Hultsch, C, Bergmann, R, Johannsen, B, and Henle, T. Appl. Radiat. Isot., 59, 43-48 (2003); Ding, Y. S, Shiue, C. Y, Fowler, J. S, Wolf, A. P, and Plenevaux, A. J. Fluorine Chem., 48, 189-205 (1990).

Scheme 3 illustrates a generally applicable synthetic route for generating a compound according to Formula I and subsequent radiolabeling of this compound with ¹⁸F in order to arrive at a compound according to Formula II. Scheme 3 depicts the formation of an O- benzotriazolyl substituted aromatic moiety connected to a peptide, compound 1, which is to be understood as a general representative of any compound according to Formula I, and subsequent direct radiolabeling towards the corresponding ¹⁸F-labelled compound **2,** which represents a compound according to Formula II. Compound **1**, containing an O-benzotriazolyl moiety is prepared by 1-hydroxybenzotriazole mediated coupling of trimethylammonium benzoic acid, compound **3**, to a resin bound protected peptide with the concomitant displacement of trimethylammonium with O-benzotriazole. Compound **1** was obtained by the cleavage from the resin according to well known methods in peptide chemistry (Chan, W. C, and White, P. D. (Editors) Fmoc Solid Phase Peptide Synthesis, Oxford University Press (2000), and references therein). The oxabenzotrizole moiety can be displaced by ¹⁸F or ¹⁹F under standard conditions (Wüst, F, Hultsch, C, Bergmann, R, Johannsen, B, and Henle, T. Appl. Radiat. Isot., 59, 43-48 (2003); Ding, Y. S, Shiue, C. Y, Fowler, J. S, Wolf, A. P, and Plenevaux, A. J. Fluorine Chem., 48, 189-205 (1990). The oxabenzotrizole moiety can also be substituted with cold fluoride. In general this method is applicable to the generation of all compounds according to Formula I and to the subsequent radiolabeling of such compounds in order to arrive at all compounds according to Formula II.

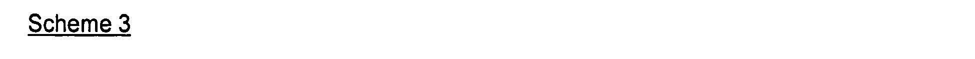

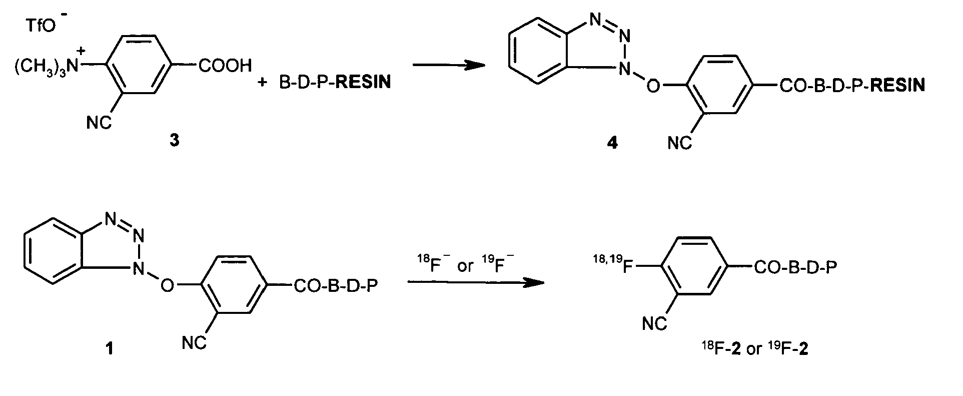

Scheme 4 depicts an alternative method for generating a compound according to Formula I. According to this method, 4-oxobenzotriazolylbenzoic acid, compound **6**, can be prepared independently, and is coupled later to the terminus of resin bound B-D-P. Compound **1**, which is to be understood as a general representative of any compound according to Formula I, was obtained by the cleavage from the resin according to well known methods in peptide chemistry. In general this method is applicable to the generation of all compounds according to Formula I.

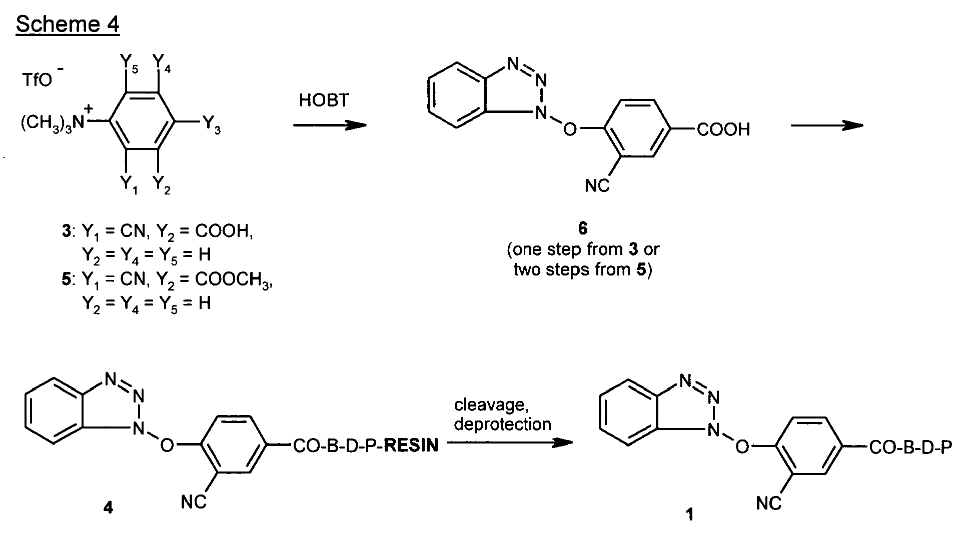

The invention also provides two other independent methods for the preparation of compounds according to Formula I. These methods are illustrated in Schemes 5 and 6. Again, these methods are applicable to the generation of all compounds according to Formula I.

The intermediate **6** can also be prepared from the corresponding boronic acids **7** by copper promoted displacement, according to e.g. the general method described in Lam, P. Y. S, Charles, G, Clark, C. G, Saubern, S, Adams, J, Kristin, M, Averill, K. M, Chan, M. T, Combs, A. "Copper Promoted Aryl/Saturated Heterocyclic C-N Bond Cross-Coupling with Arylboronic Acid and Arylstannane" SynLett., 5, 674 (2000).

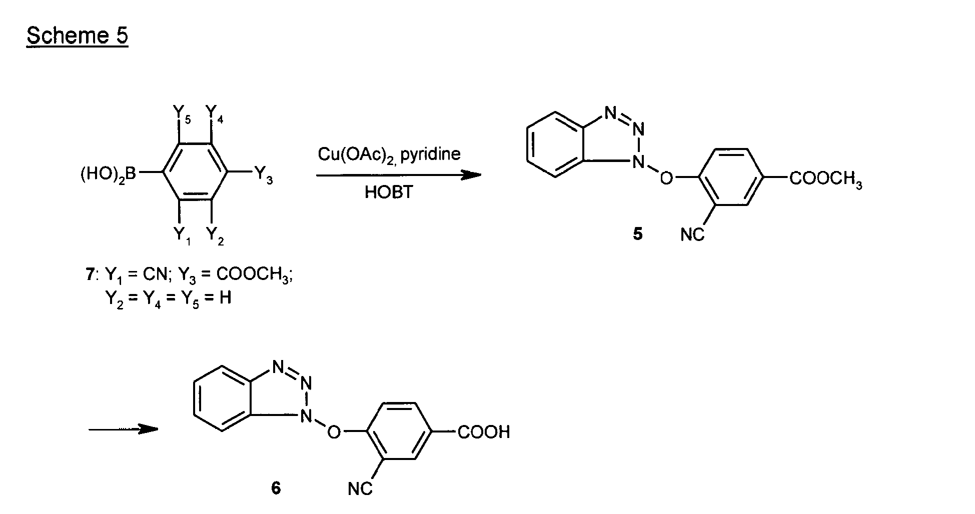

Compound **6** is converted to compound **1**, which is to be understood as a general representative of any compound according to Formula I, as shown in scheme 4.

Compound **1**, which is to be understood as a general representative of any compound according to Formula I, can also be prepared in solid phase as shown in Scheme 6.

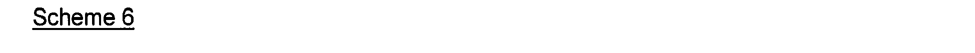

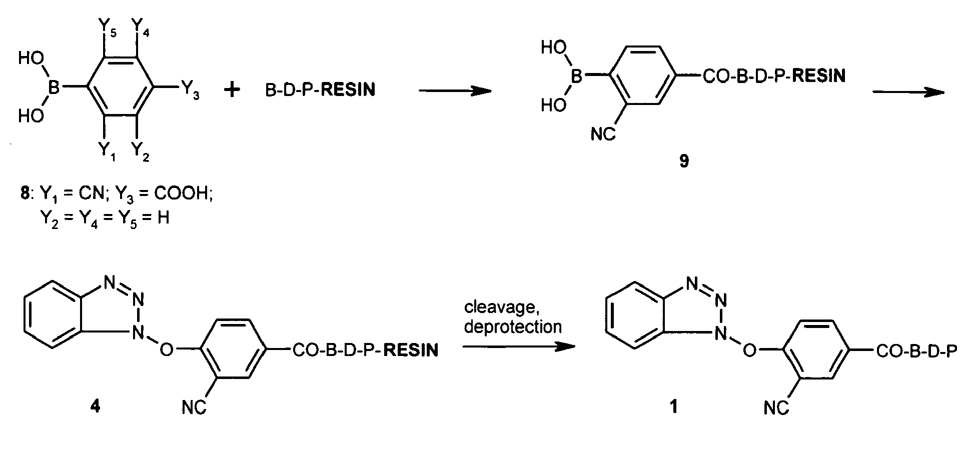

### EXAMPLES:

### General

Peptide synthesis was carried out using Rink-Amide resin (0,68 mmol/g) following standard Fmoc strategy (Fields GB, Noble RL. Solid phase peptide synthesis utilizing 9-fluorenylmethoxycarbonyl amino acids. Int. J. Pept. Protein Res. 1990; 35: 161-214). All amino acid residues are, if not further specified, L-amino acid residues.

### Fmoc-deprotection (general procedure)

The resin-bound Fmoc peptide was treated with 20% piperidine in DMF (v/v) for 5 min and a second time for 20 min. The resin was washed with DMF (2×), CH₂Cl₂ (2×), and DMF (2×).

### HBTU/HOBT coupling (general procedure)

A solution of Fmoc-Xaa-OH (4 eq), HBTU (4 eq), HOBT (4 eq), DIEA (4 eq) in DMF was added to the resin-bound free amine peptide and shaken for 90 min at room temperature. The coupling was repeated for another 60 min and the resin was washed with DMF (2x), CH₂Cl₂ (2x), and DMF (2×).

### Radiolabeling (general procedure)

No-carrier-added aqueous [¹⁸F]fluoride ion was produced by irradiation of [¹⁸O]H₂O via the ¹⁸O(p,n)¹⁸F nuclear reaction. Resolubilization of the aqueous [¹⁸F]fluoride (500-1500 MBq) was accomplished by filtration through a QMA SepPak which was preconditioned with 5 ml 0,5M K₂CO₃, washed with 5 ml water, and dried by pushing through air. 100 µl of the ¹⁸F were passed through the SepPak and dried by pushing through air. The ¹⁸F was eluted into a conical vial with 4 ml Kryptofix 2.2.2^{®}/MeCN/K₂CO₃/water mixture. The resulting solution (50-500 MBq) was dried azeotropically four times in an N₂ stream at 120 °C. To the vial containing anhydrous [¹⁸F]fluoride was added the fluorination precursor (1-4 mg) in DMSO (300-500 µl). After incubation at 50-70 °C for 15-60 min, the crude reaction mixture is analyzed using an analytical HPLC (Column Zorbax SB C18, 50×4.6mm, 1.8µ, 2ml/min, solvent A: H₂O, solvent B: MeCN, gradient: 5%-95% B in 7 min or Column Econosphere C18 , 53×7mm, 3µ, 3ml/min (Alltech), solvent A: H₂O + 0.1%TFA, solvent B: MeCN/H₂O 9/1 + 0.1 % TFA, gradient: 5-95% B in 7 min).

### Synthesis and labeling of 4-(Benzotriazol-1-yloxy)-3-cyano-benzoyl-valyl-β-alanyl-phenylalanyl-glycine amide (1a, Example 1, cf. scheme 3):

4-(Benzotriazol-1-yloxy)-3-cyano-benzoyl-valyl-β-alanyl-phenylalanyl-glycine amide was synthesized from the corresponding resin bound tetrapeptide and (4-Carboxy-2-cyanophenyl)-trimethyl-ammonium trifluoromthanesulfonate followed by cleavage and deprotection as shown below.

The peptide was fluorinated with [¹⁸F]potassium fluoride in the presence of K₂CO₃ and Kryptofix 2.2.2^{®} in DMSO to yield ¹⁸F-labelled peptide.

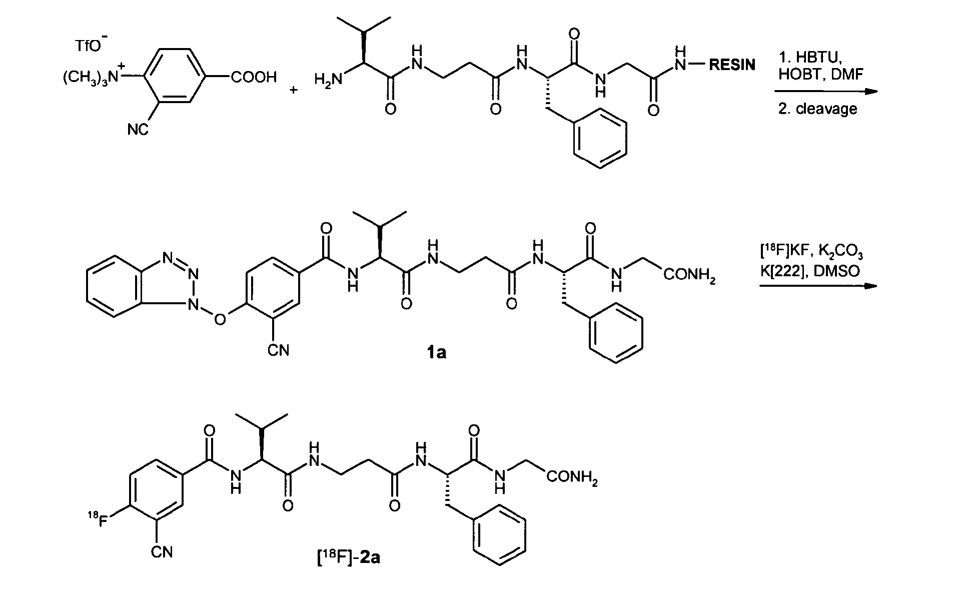

The resin-bound tetrapeptide was prepared according to the above described general procedures. The solution of (4-Carboxy-2-cyano-phenyl)-trimethyl-ammonium trifluoromthanesulfonate (4 eq), HBTU (4 eq), HOBT (4 eq) and DIPEA (4 eq) in DMF was added to the resin-bound free amine tetrapeptide and shaken for 4 h at ambient temperature. The resin was washed with DMF (4×) and CH₂Cl₂ (4×) and dried in vacuum. The peptide was cleaved from resin by treatment with a mixture of TFA, water, phenol and triisopropylsilane (85:5:5:5 v-%). The peptide was then precipitated with methyl-*tert*-butyl ether, the solvent was removed by centrifugation, and the crude product was purified by RP-HPLC. The purified product (1a) was analyzed by RP-HPLC (5-95 % acetonitrile / 12 min): tᵣ = 6.72 min, and ESI-MS: *m*/*z* = 654.2 (M+H)⁺.

Labeling was performed according to the above described general procedure. The F-18 labeled product ([¹⁸F]-**2a**) is confirmed by co-injection with the non-radioactive F-19 fluoro standard [¹⁹F]-**2a** on the Econsphere analytical HPLC.

### Synthesis of 3-Cyano-4-fluoro-benzoyl-valyl-β-alanyl-phenylalanyl-glycine amide (F-19 fluoro standard [¹⁹F]-2a)

The resin-bound tetrapeptide (H-valyl-β-alanyl-phenylalanyl-glycinyl-Rink amide resin) was prepared according to the above described general procedures. The solution of 3-Cyano-4-fluoro-benzoic acid (4 eq), HBTU (4 eq), HOBT (4 eq) and DIPEA (4 eq) in DMF was added to the resin-bound free amine tetrapeptide and shaken for 4 h at ambient temperature. The resin was washed with DMF (4×) and CH₂Cl₂ (4×) and dried in vacuum. The peptide was cleaved from resin by treatment with a mixture of TFA, water, phenol and triisopropylsilane (85:5:5:5 v-%). The peptide was then precipitated with methyl-*tert*-butyl ether, the solvent was removed by centrifugation, and the crude product was purified by RP-HPLC. The purified product ([¹⁹F]-**2a**) was analyzed by RP-HPLC (5-95 % acetonitrile / 12 min): tᵣ = 6.03 min, and ESI-MS: *m*/*z* = 539.1 (M+H)⁺.

### Synthesis and labeling of 4-(Benzotriazol-1-yloxy)-3-cyano-benzoyl-valyl-β-alanyl-histidyl(π-Me)-glycine amide (1b, Example 2, cf. scheme 3):

4-(Benzotriazol-1-yloxy)-3-cyano-benzoyl-valyl-β-alanyl-histidyl(π-Me)-glycine amide was synthesized from the corresponding resin bound tetrapeptide and (4-Carboxy-2-cyano-phenyl)-trimethyl-ammonium trifluoromthanesulfonate followed by cleavage and deprotection as shown below.

The peptide was fluorinated with [¹⁸F]potassium fluoride in the presence of K₂CO₃ and Kryptofix 2.2.2^{®} in DMSO to yield ¹⁸F-labelled peptide.

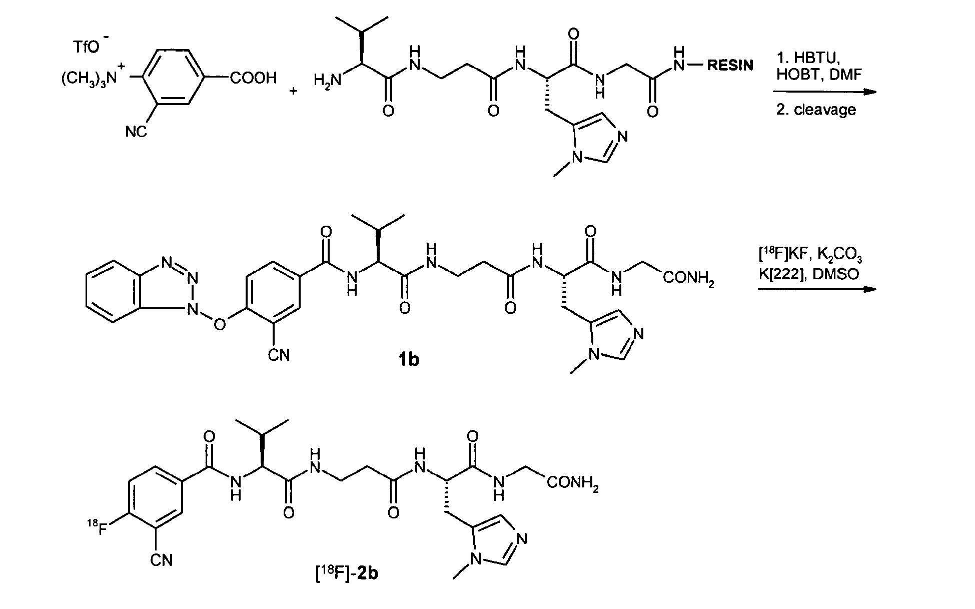

The resin-bound tetrapeptide was prepared according to the above described general procedures. The solution of (4-Carboxy-2-cyano-phenyl)-trimethyl-ammonium trifluoromthanesulfonate (4 eq), HBTU (4 eq), HOBT (4 eq) and DIPEA (4 eq) in DMF was added to the resin-bound free amine tetrapeptide and shaken for 12 h at ambient temperature. The resin was washed with DMF (4×) and CH₂Cl₂ (4×) and dried in vacuum. The peptide was cleaved from resin by treatment with a mixture of TFA, water, phenol and triisopropylsilane (85:5:5:5 v-%). The peptide was then precipitated with methyl-*tert*-butyl ether, the solvent was removed by centrifugation, and the crude product was purified by RP-HPLC. The purified product (**1b**) was analyzed by RP-HPLC (5-95 % acetonitrile / 12 min): tᵣ = 5.22 min, and ESI-MS: *m*/*z* = 658.1 (M+H)⁺.

Labeling was performed according to the above described general procedure. The F-18 labeled product ([¹⁸F]-**2b**) is confirmed by co-injection with the non-radioactive F-19 fluoro standard ([¹⁹F]-**2b**) on the Econsphere analytical HPLC.

### Synthesis of 3-Cyano-4-fluoro-benzoyl-valyl-β-alanyl-histidyl(π-Me)-glycine amide (F-19 fluoro standard [¹⁹F]-2b):

The resin-bound tetrapeptide (H-valyl-β-alanyl-histidyl(π-Me)-glycinyl-Rink amide resin) was prepared according to the above described general procedures. The solution of 3-Cyano-4-fluoro-benzoic acid (4 eq), HBTU (4 eq), HOBT (4 eq) and DIPEA (4 eq) in DMF was added to the resin-bound free amine tetrapeptide and shaken for 4 h at ambient temperature. The resin was washed with DMF (4×) and CH₂Cl₂ (4×) and dried in vacuum. The peptide was cleaved from resin by treatment with a mixture of TFA, water, phenol and triisopropylsilane (85:5:5:5 v-%). The peptide was then precipitated with methyl-*tert*-butyl ether, the solvent was removed by centrifugation, and the crude product was purified by RP-HPLC. The purified product ([¹⁹F]-**2b**) was analyzed by RP-HPLC (5-95 % acetonitrile / 12 min): tᵣ = 4.45 min, and ESI-MS: *m*/*z* = 543.1 (M+H)⁺.

### Synthesis and labeling of 3-Cyano-4-([1,2,3]triazolo[4,5-b]pyridin-3-yloxy)-benzoyl-(5-aminopentanoyl)-phenylalanyl-(4(S)-amino-3(S)-hydroxy-6-methyl)heptanoyl-leucine amide (10, Example 3, cf. scheme 3):

3-Cyano-4-([1,2,3]triazolo[4,5-b]pyridin-3-yloxy)-benzoyl-(5-aminopentanoyl)-phenylalanyl-(4(S)-amino-3(S)-hydroxy-6-methyl)heptanoyl-leucine amide was synthesized from the corresponding resin bound tetrapeptide and (4-Carboxy-2-cyanophenyl)-trimethyl-ammonium trifluoromthanesulfonate followed by cleavage and deprotection as shown below. The peptide was fluorinated with [¹⁸F]potassium fluoride in the presence of K₂CO₃ and Kryptofix 2.2.2^{®} in DMSO to yield ¹⁸F-labelled peptide.

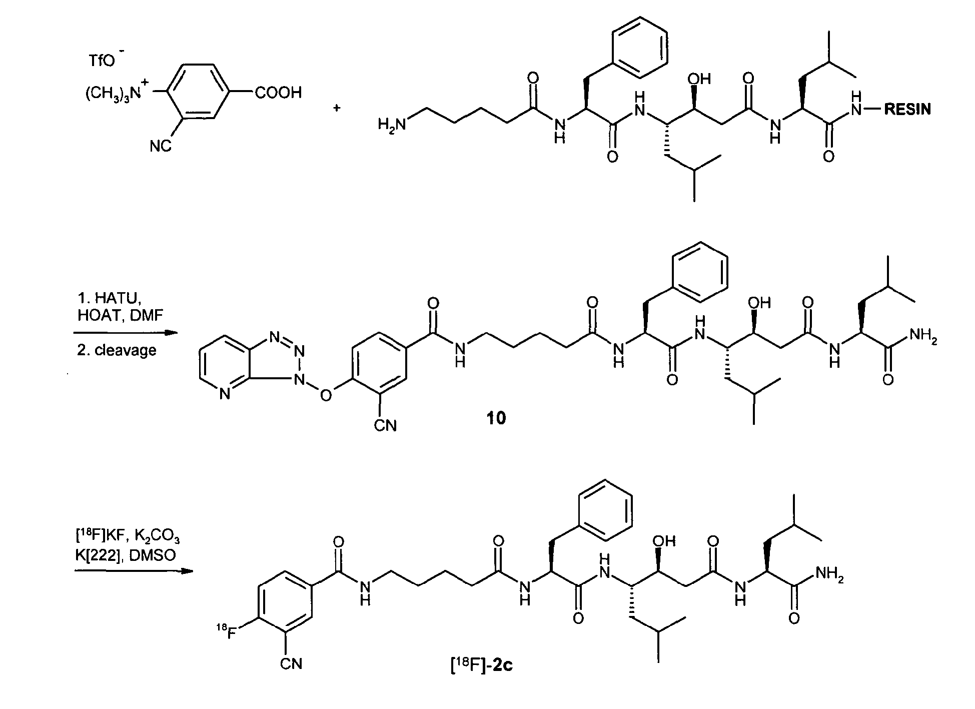

The resin-bound tetrapeptide was prepared according to the above described general procedures. The solution of (4-Carboxy-2-cyano-phenyl)-trimethyl-ammonium trifluoromthanesulfonate (4 eq), HATU (4 eq), HOAT (4 eq) and DIPEA (4 eq) in DMF was added to the resin-bound free amine tetrapeptide and shaken for 12 h at ambient temperature. The resin was washed with DMF (4×) and CH₂Cl₂ (4×) and dried in vacuum. The peptide was cleaved from resin by treatment with a mixture of TFA, water, phenol and triisopropylsilane (85:5:5:5 v-%). The peptide was then precipitated with methyl-*tert*-butyl ether, the solvent was removed by centrifugation, and the crude product was purified by RP-HPLC. The purified product (10) was analyzed by RP-HPLC (5-95 % acetonitrile /12 min): tᵣ = 6.33 min, and ESI-MS: m/z = 797.4 (M+H)⁺.

Labeling was performed according to the above described general procedure. The F-18 labeled product ([¹⁸F]-**2c**) is confirmed by co-injection with the non-radioactive F-19 fluoro standard ([¹⁹F]-**2c**) on the Econsphere analytical HPLC.

### Synthesis of 3-Cyano-4-fluoro-benzoyl-(5-aminopentanoyl)-phenylalanyl-(4(S)-amino-3(S)-hydroxy-6-methyl)heptanoyl-leucine amide (F-19 fluoro standad [¹⁹F]-2c):

The resin-bound tetrapeptide (H-(5-aminopentanoyl)-phenylalanyl-(4(S)-amino-3(S)-hydroxy-6-methyl)heptanoyl-leucinyl-Rink amide resin) was prepared according to the above described general procedures. The solution of 3-Cyano-4-fluoro-benzoic acid (4 eq), HBTU (4 eq), HOBT (4 eq) and DIPEA (4 eq) in DMF was added to the resin-bound free amine tetrapeptide and shaken for 4 h at ambient temperature. The resin was washed with DMF (4×) and CH₂Cl₂ (4×) and dried in vacuum. The peptide was cleaved from resin by treatment with a mixture of TFA, water, phenol and triisopropylsilane (85:5:5:5 v-%). The peptide was then precipitated with methyl-*tert-*butyl ether, the solvent was removed by centrifugation, and the crude product was purified by RP-HPLC. The purified product ([¹⁹F]-**2c**) was analyzed by RP-HPLC (5-95 % acetonitrile / 12 min): tᵣ = 6.35 min, and ESI-MS: *m*/*z* = 681.1 (M+H)⁺.

### Synthesis of 4-(Benzotriazol-1-yloxy)-3-cyano-benzoic acid methyl ester (11, Example 4, cf. scheme 4):

4-(Benzotriazol-1-yloxy)-3-cyano-benzoic acid methyl ester was synthesized from the (2-Cyano-4-methoxycarbonyl-phenyl)-trimethyl-ammonium trifluoromthanesulfonate as shown below.

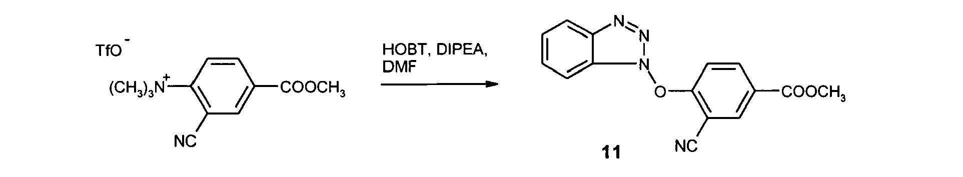

3-Cyano-4-(trimethylammonium)benzoic acid methylester trifluoromthanesulfonate, HOBT and DIPEA and were dissolved in DMF and stirred for 8 h. The solvent was removed and the residue was purified by RP-HPLC. The purified product (11) was analyzed by RP-HPLC (5-95 % acetonitrile / 12 min): tᵣ = 8.62 min, and ESI-MS: *m*/*z* = 295.0 (M+H)⁺.

### Synthesis of 4-(Benzotriazol-1-yloxy)-3-chloro-benzoyl-valyl-β-alanyl-phenylalanyl-glycine amide (12, example 5, cf. scheme 6):

4-(Benzotriazol-1-yloxy)-3-chloro-benzoyl-valyl-β-alanyl-phenylalanyl-glycine amide was synthesized from the corresponding resin bound tetrapeptide and 2-chloro-4-carboxyphenylboronic acid followed by copper-mediated displacement of the boronic acid moiety with HOBT and subsequent cleavage as shown below.

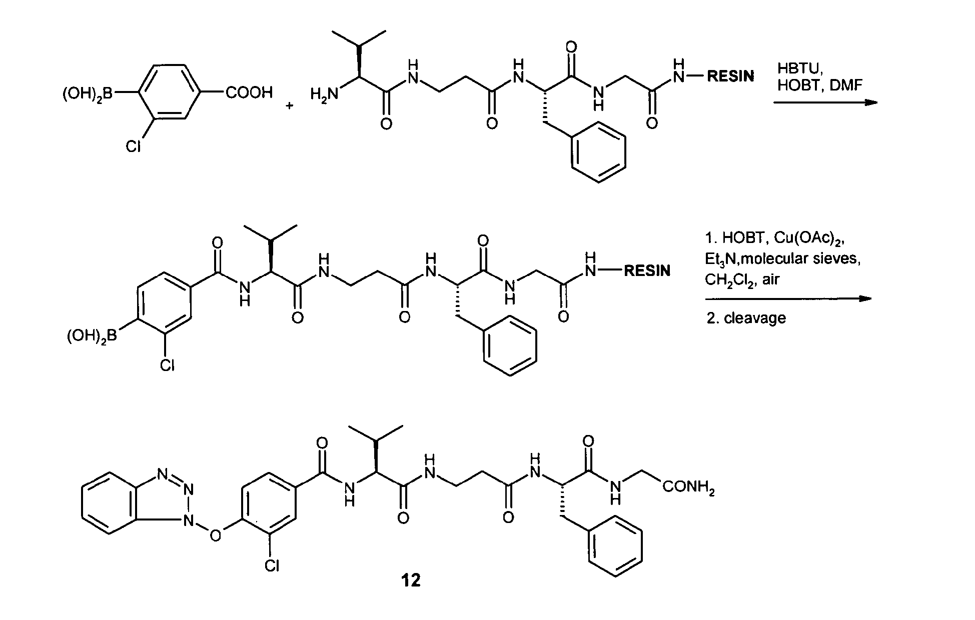

The resin-bound tetrapeptide was prepared according to the above described general procedures. The boronic acid derivative (4 eq) was solved in DMF together with HBTU (4 eq), HOBT (4 eq) and DIPEA (4 eq). The solution was shaken with the resin-bound tetrapeptide for 4 h. The resin was then washed with DMF (4×) and CH₂Cl₂ (4×). The resin was then shaken with solution of HOBT (4 eq), copper(II)acetate (6 eq) and triethylamine (8 eq) in CH₂Cl₂, and 4A molecular sieves for 48 h at ambient temperature. During the reaction the solution was exposed to air. The resin was then washed with DMF (4×) and CH₂Cl₂ (4×) and dried *in vacuo.* Cleavage of the product from resin was achieved by treatment with TFA/water (80 : 20 v-%) for 2 h. The product was precipitated with methyl-*tert*-butyl ether, the solvent was removed by centrifugation, and the crude product was purified by RP-HPLC. The purified product (12) was analyzed by RP-HPLC (5-95 % acetonitrile / 12 min): tᵣ = 5.79 min and ESI-MS: *m*/*z* = 663.2 (M+H)⁺.

For the following procedures, LG is selected from the group of 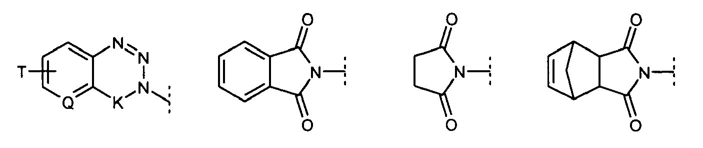 wherein T is H or Cl, Q is CH or N, K is absent or C=O, according to Formula I.

### Procedure for the displacement of the trimethylamino group by a N-hydroxy-type leaving group (LGOH):

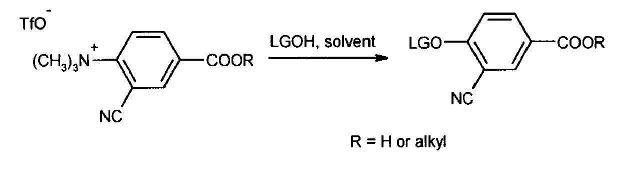

3-cyano-4-(trimethylammonio)benzoic acid or a corresponding alkyl ester thereof is solved in DMF, DMSO, acetonitrile, DMPU or any solvent suitable for a nucleophilic aromatic substitution reaction. To this solution is added the N-hydroxy-type leaving group according to the above definition. A base like tertiary amine (triethylamine, DIPEA), potassium carbonate, or sodium hydride or a comparable base may be added. The solution is then stirred at ambient temperature, elevated temperature or under microwave conditions. The product is obtained after removal of the solvent and purification of the crude by reversed phase or normal phase chromatography.

### Procedure for the displacement of a boronic acid group by a N-hydroxy-type leaving group (LGOH):

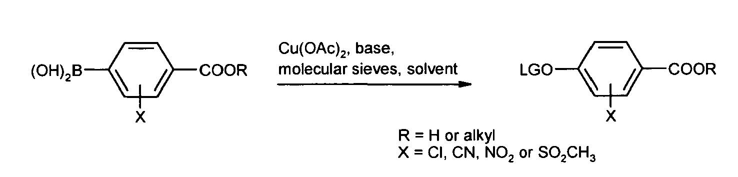

The substituted 4-carboxyphenylboronic acid or a corresponding alkylcarboxylic ester thereof is solved in either CH₂Cl₂, DMF, DMSO, acetonitrile, DMPU or mixtures thereof. To this solution is added the N-hydroxy-type leaving group according to the above definition, an amine base like triethylamine, DIPEA or pyridine, copper(II)acetate or a comparable copper salt, and molecular sieves. Ionic liquid (BMI or related) maybe added. The solution is then stirred at ambient temperature in the presence of air or molecular oxygen. Alternatively the reaction can be carried out using an oxidative agent like TEMPO, possibly under elevated temperature. The product is obtained after removal of the solvent and purification of the crude by reversed phase or normal phase chromatography.

### Procedure for the saponification of 3-cvano-4-(LGO)-benzoic acid alkyl esters:

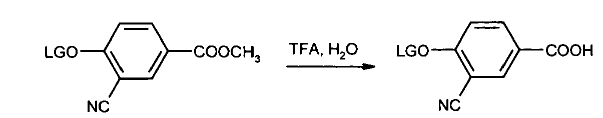

The alkyl ester is treated with a mixture of TFA and water under ambient or elevated temperature. Subsequently, the solvent is removed and the crude benzoic acid is purified by normal phase or reversed phase chromatography. The benzoic acid derivative is coupled to a resin-bound free amine peptide using one of various standard coupling conditions known in the literature.

### Analytical data for non-radioactive compounds

Compounds were analyzed on a Purosher® C-18, 4x125mm, 5µm pore size, 1ml/min, solvent A: H₂O + 0.1%TFA, solvent B: MeCN + 0.1%TFA, gradient: 5-95% B in 12 min. Products were confirmed by ESI-MS. Purity was assessed by UV (215 nm). The following table summarizes retention times and observed ESI-MS signals of the shown compounds.

| **Preparative Example** | **Retention Time** | **[M+H]⁺** |
|---|---|---|
| 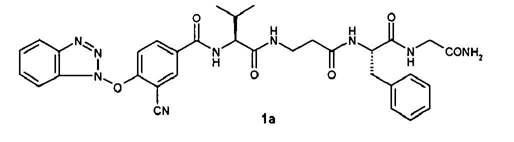 | 6.72 min | 654,2 |
| 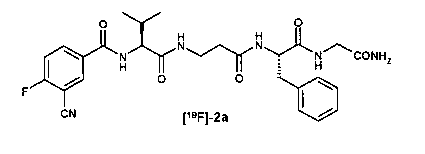 | 6.03 min | 539.1 |
| 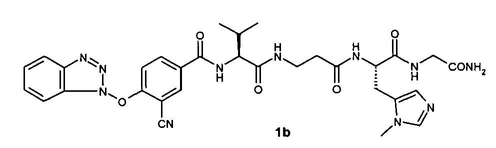 | 5.22 min | 658,1 |
| 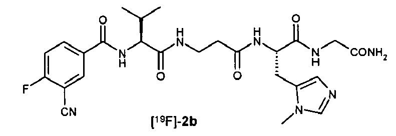 | 4.45 min | 543.1 |
| 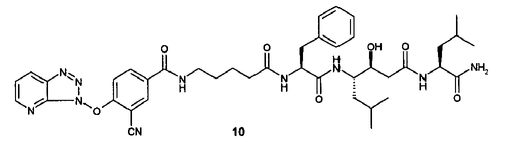 | 6.33 min | 797.4 |
| 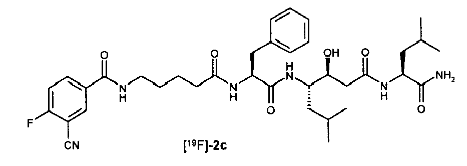 | 6.35 min | 681.1 |
| 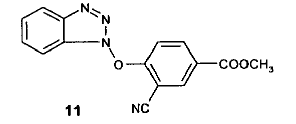 | 8.62 min | 295.0 |
| 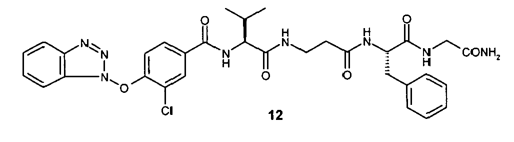 | 5.79 min | 663.2 |

### Analysis of F-18-fluorinated compounds and comparison with labelling of the corresponding trimethylammonium precursor

The identity of F-18 radiolabelled products is confirmed by coinjection with the non-radioactive F-19 fluoro standard on the Econospher analytical HPLC (see general procedure for radiolabeling).
Fig. 1 shows the radiotrace of the crude reaction mixture after incubating precursor **1a** and "F-18" according to the above described general procedure for radiolabeling for 60 min.
Fig. 2 shows the radiotrace of the crude reaction mixture after incubating precursor **13** and "F-18" according to the above described general procedure for radiolabeling for 60 min for comparison. 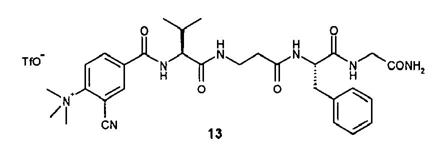
Fig. 3 shows radio- and UV-trace of the reaction according to Fig. 1 coinjected with the F-19 fluoro standard [¹⁹F]-**2a**.
Fig. 4 shows radio- and UV-trace of the reaction according to Fig. 2 coinjected with the F-19 fluoro standard [¹⁹F]-**2a**.

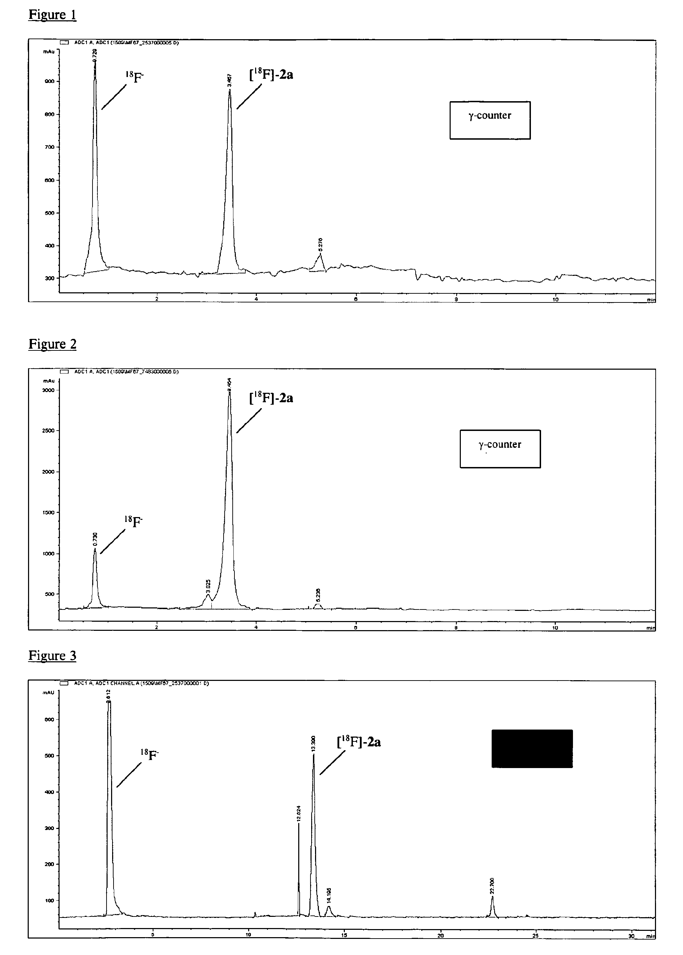

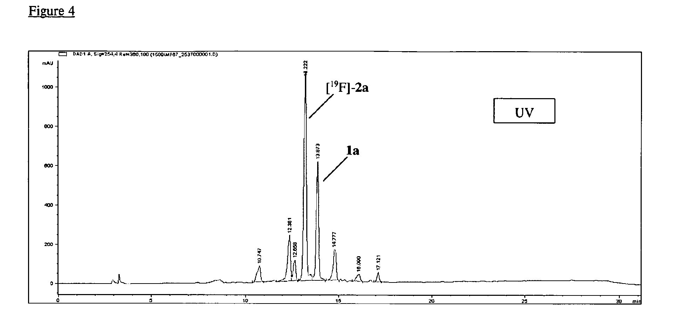

Figures 1 and 2 are superposable for the F-18-2a pic. The same is observed for Figures 3 and 4.

## Claims

1. Compound according to Formula I: 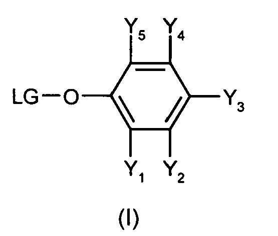 , wherein
Y₁, Y₂, Y₃, Y₄, and Y₅ are independently from each other H, CN, Cl, F, CF₃, NO₂, COCH₃, or SO₂CH₃, with the proviso that one residue of Y₁, Y₂, Y₃, Y₄, and Y₅ is A-B-D-P, wherein
A is CO or SO₂,
B is NH or NR, wherein R is a branched, cyclic or linear C₁ to C₆ alkyl group, preferably CH₃ or C₂H₅,
D is (CH₂)ₚ-CO with p being an integer between 1 and 10, or (CH₂-CH₂-O)_{q}-CH₂-CH₂-CO with q being an integer between 1 and 5, or
B-D together is a bond or one amino acid or an amino acid sequence with two (2) to twenty (20) amino acid residues,
P is a targeting agent, and
LG is a leaving group, suitable for displacement by means of a nucleophilic aromatic substitution reaction
and pharmaceutically acceptable salt, hydrate or solvate thereof.

2. Compound according to claim 1 wherein LG is selected from the group of 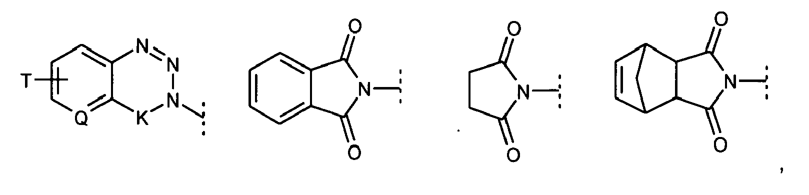 wherein,
T is H, or Cl,
Q is CH, or N,
K is absent, or C=O.

3. Compound according to claim 2 wherein LG is selected from the group of

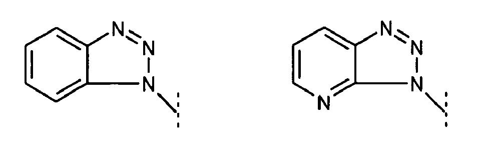

4. Compound according to claim 1 wherein Y₁, Y₂, Y₃, Y₄, and Y₅ are independently from each other selected from the group of H, CN and Cl.

5. Compound according to claim 4 wherein one of Y₁ or Y₅ is CN or Cl.

6. Compound according to Formula II: 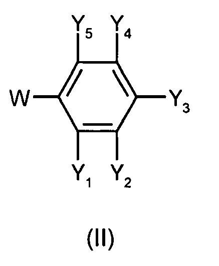 , wherein
W is ¹⁸F or ¹⁹F,
Y₁, Y₂, Y₃, Y₄, and Y₅ are independently from each other H, CN, Cl, F, CF₃, NO₂, COCH₃, or SO₂CH₃, with the proviso that one residue of Y₁, Y₂, Y₃, Y₄, and Y₅ is A-B-P, wherein
A is CO or SO₂,
B is NH or NR, wherein R is a branched, cyclic or linear C₁ to C₆ alkyl group, preferably CH₃ or C₂H₅
D is (CH₂)ₚ-CO with p being an integer between 1 and 10, or (CH₂-CH₂-O)_{q}-CH₂-CH₂-CO with q being an integer between 1 and 5, or
B-D together is a bond or one amino acid or an amino acid sequence with two (2) to twenty (20) amino acid residues, and
P is a targeting agent
and pharmaceutically acceptable salt, hydrate or solvate thereof.

7. Compound according to claim 6 wherein Y₁, Y₂, Y₃, Y₄, and Y₅ are independently from each other selected from H, CN and Cl.

8. Compound according to claim 7 wherein one of Y₁ or Y₅ is CN or Cl.

9. Method of preparing a compound according to Formula II, wherein a compound according to Formula I is labelled with ¹⁸F or ¹⁹F.

10. Method of claim 9 comprising the step of coupling a compound of formula I with ¹⁸F or ¹⁹F and thereafter converting the compound of formula II into a pharmaceutically acceptable salt, hydrate or solvate thereof if desired.

11. A composition comprising a compound according to Formula I and a pharmaceutically acceptable carrier or diluent.

12. A composition comprising a compound according to Formula II and a pharmaceutically acceptable carrier or diluent.

13. A kit comprising a sealed vial containing a predetermined quantity of a compound according to Formula I.

14. A compound according to Formula I or II for use as medicament.

15. A compound according to Formula II for use as diagnostic imaging agent.

16. A compound according to Formula II for use as imaging agent for positron emission tomography (PET).

17. Use of a compound according to Formula I for the manufacturing of a medicament.

18. Use of a compound according to Formula II for the manufacturing of a medicament.

19. Use of a compound according to Formula I for the manufacturing of a diagnostic imaging agent.

20. Use of a compound according to Formula II for the manufacturing of a diagnostic imaging agent.

21. The use according to claim 20 for the manufacture of diagnostic imaging agent for imaging tissue at target site by the targeting agent.
